# FASCICULE DE BREVET EUROPEEN

(11) **EP 3 118 186 B1**
(45) Date de publication et mention de la délivrance du brevet: **09.02.2022**
(21) Numéro de dépôt: 16184811.4
(22) Date de dépôt: 11.12.2013
(51) Int. Cl.: C07C 51/47, C11B 3/10, C11B 3/12, C07C 51/44, B01D 15/08, A23D 9/02, C11B 7/00, C11C 1/00

(54) **INSTALLATION CHROMATOGRAPHIQUE DE PRODUCTION D ACIDES GRAS POLYINSATURES**
CHROMATOGRAFISCHE ANLAGE ZUR HERSTELLUNG VON MEHRFACH UNGESÄTTIGTEN FETTSÄUREN
CHROMATOGRAPHIC FACILITY FOR PRODUCING POLYUNSATURATED FATTY ACIDS

(43) Date de publication de la demande: 18.01.2017
(62) Demande divisionnaire de: 13306701.7
(73) Titulaire: Novasep Process, 54340 Pompey (FR)
(72) Inventeur: NICOUD, Roger- Marc, 54690 Lay-Saint-Christophe (FR); BLEHAUT, Jean, 69002 Lyon (FR)
(74) Mandataire: August Debouzy

(56) Documents cités:
- EP-A1- 2 591 778
- EP-A1- 2 801 604
- EP-A2- 0 292 846
- EP-A2- 0 340 635
- JP-A- H11 209 785
- US-A1- 2011 091 947

## Description

### DOMAINE DE L'INVENTION

La présente invention concerne un procédé chromatographique de production d'acides gras polyinsaturés, tels que l'acide eicosapentaénoïque, ainsi qu'une installation adaptée à la mise en œuvre de ce procédé.

### ARRIERE-PLAN TECHNIQUE

Les acides gras, dont les acides gras polyinsaturés (en abrégé PUFA), sont des composés biologiques particulièrement importants car ils interviennent dans de nombreux processus biologiques tels que la construction et le maintien des membranes cellulaires, la synthèse d'hormones (par exemple les prostaglandines) qui jouent un rôle dans l'agrégation plaquettaire, les processus d'inflammation et la réponse immunologique, etc.

La plupart des PUFA peuvent être synthétisés par un organisme humain, à l'exception de deux familles de PUFA qui doivent être obligatoirement apportés par l'alimentation, appelés les acides gras essentiels.

Les deux familles d'acides gras essentiels sont :
- les oméga-6, qui sont particulièrement abondant dans les huiles de noix, de tournesol, de soja, de pépins de raisins ou de maïs et les volailles grasses (tel que le canard) ;
- les oméga-3 qui sont surtout présents dans les huiles de noix, dans les végétaux tel que le colza et le lin et dans les poissons gras (tels que le saumon, le thon, la sardine, le maquereau ou le hareng). Des procédés de production d'oméga-3 utilisant des cultures de micro-algues, des levures transgéniques ou de krill ont été récemment développés.

Les oméga-3 sont des PUFA particulièrement intéressants pour leurs vertus antioxydantes. Parmi ces oméga-3, l'EPA (acide eicosapentaénoïque, C20-5ω3) et le DHA (acide docosahexaénoïque, C22-6ω3) purifiés et leurs combinaisons enrichies sont les plus utilisés comme compléments alimentaires ou médicaments pour diminuer le taux de triglycérides, les risques cardiovasculaires, améliorer la cognition ou la vision, etc.

Des études cliniques récentes ont montré que le traitement de patients ayant un taux de triglycérides supérieur à 500 ml/dL par 4 grammes par jour d'ester éthylique d'EPA à 96 % sans DHA permettait de faire baisser le taux de triglycéride, sans faire augmenter le taux de LDL (« mauvais » cholestérol), alors que le traitement avec 4 grammes par jour d'un mélange d'esters éthyliques d'EPA et de DHA, à environ 50 % et 35 % respectivement, conduisait à une augmentation du taux de LDL concomitant à la diminution des triglycérides.

Jusqu'à présent, les compléments alimentaires de PUFA utilisés, notamment les oméga-3, sont essentiellement basés sur des mélanges contenant 30 à 60 % du mélange d'EPA et DHA. Dans les méthodes de séparation utilisées à ce jour, le mélange est obtenu par transestérification des triglycérides en esters éthyliques puis par un enrichissement des oméga-3 par distillation moléculaire et/ou co-cristallisation des acides gras saturés et mono-insaturés à l'urée. Les esters éthyliques enrichis sont éventuellement reconvertis en triglycérides par voie chimique ou de préférence par voie enzymatique.

Cependant, ces procédés de séparation ne sont pas satisfaisants pour la production d'un oméga-3 tel que l'EPA, le DHA ou encore l'acide stéaridonique (SDA, C18-Sω3) à plus de 80%, voire encore à plus de 96%, notamment sous forme estérifiée.

Or, la purification des oméga-3 est délicate car ces composés comprennent plusieurs doubles liaisons carbone-carbone qui les rendent sensible à l'oxydation ou la dégradation. En présence d'oxygène et lorsqu'ils sont chauffés, ces PUFA subissent notamment des réactions d'isomérisation, d'oxydation, de peroxydation et d'oligomérisation.

Ainsi, les techniques de séparation énoncées ci-dessus permettent d'obtenir un mélange de PUFA avec un bon rendement et un degré de pureté acceptable ; mais elles ne peuvent pas être mises en œuvre pour la séparation individuelle des PUFA. Elles ne permettent donc pas de séparer des oméga-3 entre eux. En effet, la distillation moléculaire, par exemple, ne peut pas économiquement éliminer le DHA de l'EPA ou du SDA ; elle ne permet pas une séparation efficace des oméga-3 à longue chaîne de type C20 et C22. La combinaison de la clathration à l'urée et de la distillation moléculaire permet d'obtenir des mélanges d'oméga-3 à plus haute pureté, au prix d'un rendement généralement faible et d'un coût opératoire élevé, mais ne peut pas être utilisée pour la séparation des oméga-3 à longue chaîne entre eux, et de l'EPA et du DHA en particulier.

Il existe donc un besoin de fournir un procédé de purification industriel d'oméga-3 sous forme estérifiée à très haute pureté.

La chromatographie est une technique de séparation fine permettant la purification ou l'enrichissement efficace de molécules dans des conditions douces et à l'abri de la lumière et de l'air.

Cette technologie repose sur la séparation des molécules qui sont mises en contact avec une phase stationnaire avec lesquelles elles ont des interactions différentes. L'utilisation d'un ou plusieurs fluides, dits phases mobiles ou éluants, permet la percolation des différentes molécules à différentes vitesses. Ces différentes vitesses permettent de séparer physiquement les molécules et de les récolter sous forme purifiée à l'issue de procédés chromatographiques à une ou plusieurs colonnes. Les fractions purifiées sont en général concentrées, dans des conditions douces à température ambiante ou modérée, par des moyens tels que l'évaporation sous vide ou les procédés membranaires.

Dans certains cas, le produit de départ de la purification chromatographique est une huile composée d'esters d'acides gras déjà enrichie par distillation moléculaire, comprenant de préférence plus de 30 % de l'oméga-3 d'intérêt, qui a subi un traitement d'élimination des composés oxydés, soit par la dernière distillation moléculaire, soit par adsorption, de préférence sur des dérivés de silice (gel de silice, bentonite, terre de diatomée) ou sur charbon actif par exemple.

De nombreux documents décrivent l'élimination de composés oxydés dans des compositions dérivées d'huiles.

A titre d'exemple, le document EP 0682006 décrit un traitement d'huiles contenant des oméga-3 par dilution dans l'hexane et ajout de 10 à 40 % en poids de charbon actif.

Le document US 4,874,629 décrit un traitement d'huiles contenant des oméga-3 par distillation à la vapeur d'eau suivie d'une adsorption des composés polaires sur de la silice.

Le document WO 2005/049772 décrit également le traitement d'une huile riche en omega-3 par dissolution dans un solvant aprotique et contact avec un dérivé de silicium.

Le document EP 0773283 décrit le traitement d'une huile contenant un PUFA d'au moins 18 carbones par contact pendant au moins 10 minutes avec au minimum 0.1 % en poids d'une terre de diatomée préalablement traitée en milieu acide à une température de 5 à 80°C, suivi ou non par une distillation à la vapeur d'eau.

Un certain nombre de procédés chromatographiques ont par ailleurs été décrits, pour l'obtention d'oméga-3 avec une haute pureté.

Ainsi, le document US 5,719,302 décrit un procédé dans lequel les PUFA sont séparés notamment à l'aide d'un éluant supercritique (le gaz carbonique sous pression), et notamment sur lit mobile simulé (SMB pour « *Simulated Moving Bed* » selon la terminologie anglo-saxonne).

Le document US 2011/0091947 décrit un autre procédé de purification d'oméga-3 utilisant la technique de la chromatographie en lit mobile simulé. Le document décrit en particulier la succession d'une étape de transestérification enzymatique, de deux étapes de distillation moléculaire, et d'une étape de type SMB, ces trois dernières étapes permettent de séparer les produits en deux fractions par ordre de temps de rétention.

Le document WO 2011/080503 décrit la purification d'oméga-3 à partir d'un dispositif comprenant deux dispositifs chromatographiques SMB disposé en série et une zone de lavage, chaque dispositif chromatographique SMB définissant une zone de séparation et étant constitué de plusieurs colonnes. La charge à traiter est injectée dans une première zone de séparation pour obtenir un flux d'extrait et un flux de raffinat, ledit flux de raffinat comprenant les composés d'intérêts étant ensuite injecté dans une colonne de la seconde zone de séparation non adjacente à une colonne de la première zone.

Le document WO 2013/005051 décrit la purification d'oméga-3 par deux séparations chromatographiques par SMB ou AMB (« *Actual Moving Bed* »*,* c'est-à-dire lit mobile réel) en phase inversée avec un éluant hydro-organique, dans lequel les deux séparations par SMB ou AMB sont réalisées séquentiellement sur le même dispositif chromatographique, ou sur deux dispositifs différents, l'intermédiaire purifié par le premier dispositif étant introduit dans le second.

Le document WO 2013/005048 décrit la purification d'EPA à plus de 90 % de pureté par une première séparation chromatographique suivie de deux séparations chromatographiques par SMB ou AMB en phase inversée avec un éluant hydro-organique à chaque étape, l'intermédiaire purifié par la première séparation chromatographique étant introduit dans la seconde séparation chromatographique, et l'intermédiaire purifié par la deuxième séparation chromatographique étant introduit dans la troisième séparation chromatographique.

Dans les deux documents précédents, l'EPA purifié est de préférence obtenu dans l'extrait de la dernière séparation ; ainsi la dernière séparation sépare l'EPA des impuretés moins retenues collectées au raffinat (comme il est bien connu de l'homme de l'art en chromatographie en phase inversée). De telles impuretés moins retenues que l'EPA peuvent être par exemple des PUFA à chaîne plus courte comme le SDA, ou des composés d'oxydation tels que des peroxydes ou des aldéhydes.

Le document EP0292846 décrit un procédé d'extraction d'esters d'EPA et DHA à partir d'huiles de poisson, par transestérification avec de l'éthanol et de l'acide sulfurique et distillation moléculaire en deux étapes.

Le document EP2801604 décrit un procédé de récupération d'un premier PUFA à partir d'un mélange comprenant le premier PUFA et au moins un deuxième acide gras. Le procédé décrit dans ce document comprend au moins une étape principale de chromatographie permettant d'obtenir un flux enrichi en premier PUFA et un flux enrichi en deuxième acide gras, au moins une étape de concentration effectuée sur le flux enrichi en deuxième acide gras afin d'obtenir un flux enrichi en acide gras et un deuxième flux appauvri en acide gras, et au moins une étape de recyclage du deuxième flux appauvri en acide gras.

Il existe encore un besoin de fournir un PUFA (ou un dérivé, notamment un ester de celui-ci) avec une pureté élevée, permettant son utilisation dans des compositions de médicament, à partir d'une charge multi-composés comprenant ledit PUFA. En particulier, il existe encore un besoin de fournir un PUFA (ou un dérivé, notamment un ester de celui-ci) essentiellement dépourvu de contaminants oxygénés de type peroxydes ou aldéhydes.

### RESUME DE L'INVENTION

L'invention décrit en premier lieu un procédé de purification d'un premier acide gras polyinsaturé à partir d'un mélange initial, le mélange initial comprenant au moins un deuxième acide gras en plus du premier acide gras polyinsaturé, le procédé comprenant :
- au moins une étape de séparation chromatographique en phase liquide du premier acide gras polyinsaturé et du deuxième acide gras, permettant de récupérer d'une part un flux enrichi en premier acide gras polyinsaturé et d'autre part un flux enrichi en deuxième acide gras ;
- une étape de traitement du flux enrichi en premier acide gras polyinsaturé, conduisant à une diminution de l'indice de peroxyde et / ou de l'indice d'anisidine de ce flux.

Selon un mode de réalisation, l'étape de traitement est une étape de distillation moléculaire.

Selon un mode de réalisation, l'étape de traitement est une étape de mise en contact avec un substrat d'adsorption, qui est de préférence choisi parmi la silice, l'alumine, le charbon actif et les dérivés de ceux-ci ; ladite mise en contact étant de préférence effectuée sans dilution du premier acide gras polyinsaturé et sans ajout d'un solvant.

Selon un mode de réalisation, le flux enrichi en premier acide gras insaturé présente, après l'étape de traitement, un indice de peroxyde inférieur ou égal à 10, de préférence inférieur ou égal à 5, ou inférieur ou égal à 2, ou inférieur ou égal à 1 ; et/ou un indice d'anisidine inférieur ou égal à 20, de préférence inférieur ou égal à 10, ou inférieur ou égal à 5, ou inférieur ou égal à 3 ; et/ou le flux enrichi en premier acide gras insaturé présente, avant l'étape de traitement, un indice de peroxyde supérieur ou égal à 1, de préférence supérieur ou égal à 2, ou supérieur ou égal à 4, ou supérieur ou égal à 6, et/ou un indice d'anisidine supérieur ou égal à 1, de préférence supérieur ou égal à 2, ou supérieur ou égal à 4, ou supérieur ou égal à 6.

Selon un mode de réalisation :
- le premier acide gras polyinsaturé est l'acide eicosapentaénoïque, et est de préférence récupéré à l'issue du procédé avec une pureté supérieure ou égale à 80 %, ou à 90 %, ou à 96 % ; ou
- le premier acide gras polyinsaturé est l'acide docosahexaénoïque, et est de préférence récupéré à l'issue du procédé avec une pureté supérieure ou égale à 70 %, ou à 80 %, ou à 90 %, ou à 95 % ; ou
- le premier acide gras polyinsaturé est l'acide arachidonique, et est de préférence récupéré à l'issue du procédé avec une pureté supérieure ou égale à 70 %, ou à 80 %, ou à 90 %, ou à 95 % ; ou
- le premier acide gras polyinsaturé est l'acide docosapentaénoïque, et est de préférence récupéré à l'issue du procédé avec une pureté supérieure ou égale à 70 %, ou à 80 %, ou à 90 %, ou à 95 %.

Selon un mode de réalisation, l'étape de séparation chromatographique du premier acide gras polyinsaturé et du deuxième acide gras est mise en œuvre avec un éluant hydro-organique.

Selon un mode de réalisation, le deuxième acide gras est un acide gras polyinsaturé, de préférence choisi parmi l'acide docosahexaénoïque, l'acide eicosapentaénoïque, l'acide arachidonique, l'acide docosapentaénoïque et l'acide stéaridonique.

Selon un mode de réalisation, le mélange initial comprend en outre un troisième acide gras, et le procédé comprend une étape de séparation chromatographique en phase liquide du premier acide gras polyinsaturé et du troisième acide gras, permettant de récupérer d'une part un flux enrichi en premier acide gras polyinsaturé et d'autre part un flux enrichi en troisième acide gras ; cette étape de séparation chromatographique étant de préférence effectuée avec un éluent hydro-organique ; et cette étape de séparation chromatographique étant de préférence effectuée en amont de l'étape de séparation chromatographique en phase liquide du premier acide gras polyinsaturé et du deuxième acide gras.

Selon un mode de réalisation, le mélange initial comprend en outre un quatrième acide gras, et le procédé comprend une étape de séparation chromatographique en phase liquide du premier acide gras polyinsaturé et du quatrième acide gras, permettant de récupérer d'une part un flux enrichi en premier acide gras polyinsaturé et d'autre part un flux enrichi en quatrième acide gras ; cette étape de séparation chromatographique étant de préférence effectuée avec un éluent hydro-organique ; et cette étape de séparation chromatographique étant de préférence effectuée en amont de l'étape de séparation chromatographique en phase liquide du premier acide gras polyinsaturé et du deuxième acide gras.

Selon un mode de réalisation :
- le premier acide gras polyinsaturé est moins retenu que le deuxième acide gras lors de l'étape de séparation chromatographique du premier acide gras polyinsaturé et du deuxième acide gras ; et/ou le premier acide gras polyinsaturé est moins retenu que le troisième acide gras lors de l'étape de séparation chromatographique du premier acide gras polyinsaturé et du troisième acide gras ; et/ou le premier acide gras polyinsaturé est moins retenu que le quatrième acide gras lors de l'étape de séparation chromatographique du premier acide gras polyinsaturé et du quatrième acide gras ;
- de préférence, le premier acide gras polyinsaturé est moins retenu que deux acides gras choisis parmi le deuxième acide gras, le troisième acide gras et le quatrième acide gras, lors des étapes de séparation chromatographique respectives du premier acide gras polyinsaturé et du deuxième, du troisième et du quatrième acide gras ;
- de manière plus particulièrement préférée, le procédé comprend successivement (i) l'étape de séparation chromatographique en phase liquide du premier acide gras polyinsaturé et du quatrième acide gras, le premier acide gras polyinsaturé étant moins retenu que le quatrième acide gras, puis (ii) l'étape de séparation chromatographique en phase liquide du premier acide gras polyinsaturé et du troisième acide gras, le premier acide gras polyinsaturé étant moins retenu que le troisième acide gras, puis (iii) l'étape de séparation chromatographique en phase liquide du premier acide gras polyinsaturé et du deuxième acide gras, le premier acide gras polyinsaturé étant plus retenu que le deuxième acide gras.

Selon un mode de réalisation, le procédé comprend une étape de traitement préliminaire conduisant à une diminution de l'indice de peroxyde et / ou de l'indice d'anisidine par rapport au mélange initial, en amont de la ou des étapes de séparation chromatographique en phase liquide, ladite étape de traitement préliminaire étant de préférence une étape de distillation moléculaire, ou une étape de mise en contact avec un substrat d'adsorption, notamment choisi parmi la silice, l'alumine, le charbon actif et les dérivés de ceux-ci.

Ainsi, en particulier selon un objet, ci-après objet I, il est proposé un procédé de purification d'un premier acide gras polyinsaturé à partir d'un mélange initial, le mélange initial comprenant au moins un deuxième acide gras en plus du premier acide gras polyinsaturé, le procédé comprenant :
- au moins une étape de séparation chromatographique en phase liquide du premier acide gras polyinsaturé et du deuxième acide gras, permettant de récupérer d'une part un flux enrichi en premier acide gras polyinsaturé et d'autre part un flux enrichi en deuxième acide gras ;
- une étape de traitement du flux enrichi en premier acide gras polyinsaturé, conduisant à une diminution de l'indice de peroxyde et / ou de l'indice d'anisidine de ce flux.

Il est également proposé un objet, ci-après objet II, selon l'objet I et dans lequel l'étape de traitement est une étape de distillation moléculaire.

Il est également proposé un objet, ci-après objet III, selon l'objet I ou II et dans lequel l'étape de traitement est une étape de mise en contact avec un substrat d'adsorption, qui est de préférence choisi parmi la silice, l'alumine, le charbon actif et les dérivés de ceux-ci ; ladite mise en contact étant de préférence effectuée sans dilution du premier acide gras polyinsaturé et sans ajout d'un solvant.

Il est également proposé un objet, ci-après objet IV, selon l'un des objets I à III et dans lequel le flux enrichi en premier acide gras insaturé présente, après l'étape de traitement, un indice de peroxyde inférieur ou égal à 10, de préférence inférieur ou égal à 5, ou inférieur ou égal à 2, ou inférieur ou égal à 1 ; et/ou un indice d'anisidine inférieur ou égal à 20, de préférence inférieur ou égal à 10, ou inférieur ou égal à 5, ou inférieur ou égal à 3, ou inférieur ou égal à 2, ou inférieur ou égal à 1 ; et/ou dans lequel le flux enrichi en premier acide gras insaturé présente, avant l'étape de traitement, un indice de peroxyde supérieur ou égal à 1, de préférence supérieur ou égal à 2, ou supérieur ou égal à 4, ou supérieur ou égal à 6, et/ou un indice d'anisidine supérieur ou égal à 1, de préférence supérieur ou égal à 2, ou supérieur ou égal à 4, ou supérieur ou égal à 6.

Il est également proposé un objet, ci-après objet V, selon l'un des objets I à IV et dans lequel :
- le premier acide gras polyinsaturé est l'acide eicosapentaénoïque, et est de préférence récupéré à l'issue du procédé avec une pureté supérieure ou égale à 80 %, ou à 90 %, ou à 96 % ; ou
- le premier acide gras polyinsaturé est l'acide docosahexaénoïque, et est de préférence récupéré à l'issue du procédé avec une pureté supérieure ou égale à 70 %, ou à 80 %, ou à 90 %, ou à 95 % ; ou
- le premier acide gras polyinsaturé est l'acide arachidonique, et est de préférence récupéré à l'issue du procédé avec une pureté supérieure ou égale à 70 %, ou à 80 %, ou à 90 %, ou à 95 % ; ou
- le premier acide gras polyinsaturé est l'acide docosapentaénoïque, et est de préférence récupéré à l'issue du procédé avec une pureté supérieure ou égale à 70 %, ou à 80 %, ou à 90 %, ou à 95 %.

Il est également proposé un objet, ci-après objet VI, selon l'un des objets I à V et dans lequel l'étape de séparation chromatographique du premier acide gras polyinsaturé et du deuxième acide gras est mise en œuvre avec un éluant hydro-organique.

Il est également proposé un objet, ci-après objet VII, selon l'un des objets I à VI et dans lequel le deuxième acide gras est un acide gras polyinsaturé, de préférence choisi parmi l'acide docosahexaénoïque, l'acide eicosapentaénoïque, l'acide arachidonique, l'acide docosapentaénoïque et l'acide stéaridonique.

Il est également proposé un objet, ci-après objet VIII, selon l'un des objets I à VII et dans lequel le mélange initial comprend en outre un troisième acide gras, et le procédé comprend une étape de séparation chromatographique en phase liquide du premier acide gras polyinsaturé et du troisième acide gras, permettant de récupérer d'une part un flux enrichi en premier acide gras polyinsaturé et d'autre part un flux enrichi en troisième acide gras ; cette étape de séparation chromatographique étant de préférence effectuée avec un éluent hydro-organique ; et cette étape de séparation chromatographique étant de préférence effectuée en amont de l'étape de séparation chromatographique en phase liquide du premier acide gras polyinsaturé et du deuxième acide gras.

Il est également proposé un objet, ci-après objet IX, selon l'objet VIII et dans lequel le mélange initial comprend en outre un quatrième acide gras, et le procédé comprend une étape de séparation chromatographique en phase liquide du premier acide gras polyinsaturé et du quatrième acide gras, permettant de récupérer d'une part un flux enrichi en premier acide gras polyinsaturé et d'autre part un flux enrichi en quatrième acide gras ; cette étape de séparation chromatographique étant de préférence effectuée avec un éluent hydro-organique ; et cette étape de séparation chromatographique étant de préférence effectuée en amont de l'étape de séparation chromatographique en phase liquide du premier acide gras polyinsaturé et du deuxième acide gras.

Il est également proposé un objet, ci-après objet X, selon l'un des objets I à IX et dans lequel :
- le premier acide gras polyinsaturé est moins retenu que le deuxième acide gras lors de l'étape de séparation chromatographique du premier acide gras polyinsaturé et du deuxième acide gras ; et/ou le premier acide gras polyinsaturé est moins retenu que le troisième acide gras lors de l'étape de séparation chromatographique du premier acide gras polyinsaturé et du troisième acide gras ; et/ou le premier acide gras polyinsaturé est moins retenu que le quatrième acide gras lors de l'étape de séparation chromatographique du premier acide gras polyinsaturé et du quatrième acide gras ;
- de préférence, le premier acide gras polyinsaturé est moins retenu que deux acides gras choisis parmi le deuxième acide gras, le troisième acide gras et le quatrième acide gras, lors des étapes de séparation chromatographique respectives du premier acide gras polyinsaturé et du deuxième, du troisième et du quatrième acide gras ;
- de manière plus particulièrement préférée, le procédé comprend successivement (i) l'étape de séparation chromatographique en phase liquide du premier acide gras polyinsaturé et du quatrième acide gras, le premier acide gras polyinsaturé étant moins retenu que le quatrième acide gras, puis (ii) l'étape de séparation chromatographique en phase liquide du premier acide gras polyinsaturé et du troisième acide gras, le premier acide gras polyinsaturé étant moins retenu que le troisième acide gras, puis (iii) l'étape de séparation chromatographique en phase liquide du premier acide gras polyinsaturé et du deuxième acide gras, le premier acide gras polyinsaturé étant plus retenu que le deuxième acide gras.

Il est également proposé un objet, ci-après objet XI, selon l'un des objets I à X et comprenant une étape de traitement préliminaire conduisant à une diminution de l'indice de peroxyde et / ou de l'indice d'anisidine par rapport au mélange initial, en amont de la ou des étapes de séparation chromatographique en phase liquide, ladite étape de traitement préliminaire étant de préférence une étape de distillation moléculaire, ou une étape de mise en contact avec un substrat d'adsorption, notamment choisi parmi la silice, l'alumine, le charbon actif et les dérivés de ceux-ci.

L'invention a également pour objet une installation de purification d'un premier acide gras polyinsaturé à partir d'un mélange initial, l'installation comprenant :
- au moins une unité de séparation chromatographique en phase liquide du premier acide gras polyinsaturé et d'un deuxième acide gras, à laquelle sont connectées en sortie d'une part une conduite de flux enrichi en premier acide gras polyinsaturé et d'autre part une conduite de flux enrichi en deuxième acide gras ;
- au moins une unité de traitement alimentée par la conduite de flux enrichi en premier acide gras polyinsaturé, l'unité de traitement étant adaptée à effectuer une diminution de l'indice de peroxyde et / ou l'indice d'anisidine.

Selon l'invention, l'unité de traitement est une unité de distillation moléculaire ou est une unité de mise en contact avec un substrat d'adsorption, de préférence choisi parmi la silice, l'alumine, le charbon actif et les dérivés de ceux-ci ; ladite unité de mise en contact ne comportant pas d'apport de solvant.

Selon un mode de réalisation, l'installation comprend une unité de séparation chromatographique en phase liquide du premier acide gras polyinsaturé et d'un troisième acide gras, et éventuellement une unité de séparation chromatographique en phase liquide du premier acide gras polyinsaturé et d'un quatrième acide gras, lesdites unités de séparation chromatographique en phase liquide étant de préférence situées en amont de l'unité de séparation chromatographique en phase liquide du premier acide gras polyinsaturé et d'un deuxième acide gras.

Selon un mode de réalisation, au moins une unité de séparation chromatographique en phase liquide, de préférence toutes les unités de séparation chromatographique en phase liquide, sont des unités de séparation chromatographique comportant une pluralité de colonnes de séparation chromatographique, et de préférence sont des unités de séparation chromatographique en lit mobile simulé et / ou en lit mobile réel.

La présente invention permet de surmonter les inconvénients de l'état de la technique. Elle fournit plus particulièrement un procédé d'obtention d'un PUFA (ou dérivé, notamment un ester de celui-ci) de pureté élevée et susceptible d'être utilisé dans des compositions de médicament, à partir d'une charge multi-composés comprenant ledit PUFA. En particulier, le PUFA ainsi obtenu présente un faible taux, ou est essentiellement dépourvu, de contaminants oxygénés de type peroxydes ou aldéhydes.

L'invention repose sur la constatation par les inventeurs que la séparation d'un oméga-3 par une ou plusieurs étapes de chromatographie en phase liquide, réalisées à l'abri de la lumière et de l'oxygène, conduit de façon surprenante à une augmentation de l'indice de peroxyde et/ou de l'indice d'anisidine, de sorte qu'un traitement complémentaire d'élimination des composés d'oxydation est nécessaire, et ce même si la dernière étape de chromatographie sépare l'oméga-3 recherché des composés moins retenus.

Plus précisément, à chaque fois que le composé d'intérêt est moins retenu que les composés à éliminer (prélèvement au raffinat, dans le cadre d'un procédé continu), l'indice de peroxyde et/ou l'indice d'anisidine augmentent, rendant nécessaire un traitement complémentaire d'élimination des composés d'oxydation.

La présence d'une ou plusieurs étapes dans lesquelles le composé d'intérêt est plus retenu que les composés à éliminer (prélèvement à l'extrait, dans le cadre d'un procédé continu) peut réduire l'indice de peroxyde et/ou l'indice d'anisidine au cours du procédé. Toutefois, il a été constaté que cette réduction n'est pas nécessairement suffisante, rendant nécessaire un traitement complémentaire d'élimination des composés d'oxydation.

### BREVE DESCRIPTION DES FIGURES

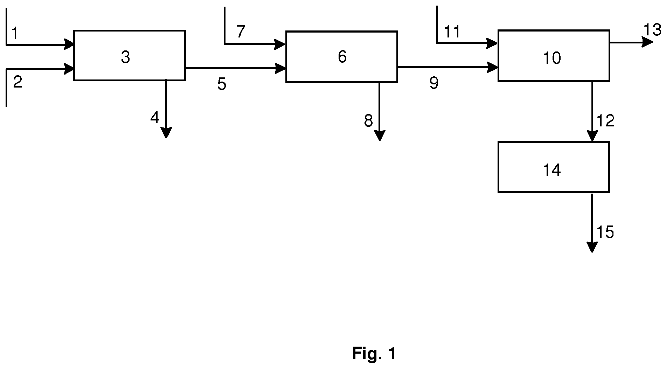

La **figure** 1 représente de manière schématique un mode de réalisation d'une installation pour la mise en œuvre de l'invention.

### DESCRIPTION DE MODES DE REALISATION DE L'INVENTION

L'invention est maintenant décrite plus en détail et de façon non limitative dans la description qui suit.

De manière générale, les proportions exprimées sont des proportions massiques, sauf mention contraire.

### Principe général du procédé

Le procédé de l'invention permet d'obtenir un premier PUFA sous forme purifiée, à partir d'un mélange initial. Le mélange initial comprend au moins un deuxième acide gras non souhaité, qui est de préférence un deuxième PUFA non souhaité, et de préférence un certain nombre d'autres acides gras non souhaités, tels que des acides gras saturés ou mono-insaturés et d'autres PUFA, ainsi que d'autres impuretés éventuelles.

Le mélange initial peut être un mélange d'acides gras dérivé de poisson, de végétaux, d'algues et/ou de levure, et de préférence de poisson. Il peut s'agir d'une matière brute, par exemple de l'huile de poisson ou de l'huile d'algue ou de l'huile de levure. Il peut également s'agir d'un produit dérivé des matières brutes ci-dessus, et par exemple dérivé d'huile de poisson, d'huile d'algue et/ou d'huile de levure. L'huile peut par exemple être extraite de végétaux, algues ou levures naturelles ou génétiquement modifiées.

Par « *produit dérivé d'une matière brute* », on entend une matière brute qui a été soumise à une ou plusieurs étapes de traitement. Ces étapes de traitement peuvent comprendre une ou plusieurs étapes de désintégration cellulaire, de broyage, de séparation ou de purification (par exemple un fractionnement) et/ou une étape d'hydrolyse pour convertir des triglycérides en acides gras libres et/ou une étape d'estérification pour convertir les acides gras en alkyl esters et/ou une étape de transestérification pour convertir les triglycérides gras en alkyl esters, et de préférence en éthyl esters, et/ou une étape de réduction de l'indice de peroxyde et/ou de l'indice d'anisidine (cf. ci-dessous), et/ou une étape de distillation moléculaire, et/ou une ou plusieurs étapes de séparation chromatographique, etc.

Selon un mode de réalisation avantageux, le mélange initial est un produit estérifié ou transestérifié, tel qu'une huile de poisson, une huile végétale, une huile d'algue ou une huile de levure transestérifiée.

Ainsi, chaque acide gras (et en particulier chaque PUFA) obtenu ou utilisé dans le procédé de l'invention peut être un dérivé d'acide gras, notamment sous la forme d'un monoglycéride, diglycéride ou triglycéride, d'un ester, d'un phospholipide, d'un amide, d'une lactone ou d'un sel.

Les formes acide gras libre et esters sont préférées, et tout particulièrement les esters. Les esters sont typiquement des alkyl esters, par exemple des alkyl esters en C1-C6, notamment en C1-C4, par exemple des méthyl esters et des éthyl esters. Les éthyl esters sont préférés.

Ainsi, le premier PUFA, le deuxième acide gras, le troisième acide gras et le quatrième acide gras mentionnés dans la présente demande peuvent être par exemple sous forme acide gras libre ou ester, et de préférence sont sous forme de composés éthyl esters.

En faisant référence à la **figure 1****,** le procédé selon l'invention peut être mis en œuvre dans une installation comprenant une première unité chromatographique 10. La première unité chromatographique 10 assure la séparation entre le premier PUFA et le deuxième acide gras.

A chaque fois qu'il est fait mention dans la présente demande d'une séparation entre le premier PUFA et un acide gras donné, il est entendu que d'autres acides gras peuvent également être séparés du premier PUFA simultanément avec la séparation vis-à-vis de l'acide gras donné.

Généralement, chaque séparation chromatographique sépare le premier PUFA d'un ensemble de composés plus polaires que lui ou moins polaires que lui. La séparation peut également s'effectuer selon des critères de taille de longueur de chaine aliphatique et de nombre d'insaturation. Plus généralement, les effets pouvant être dépendants des éluants utilisés, la séparation s'effectue selon des critères de temps de rétention qui sont différents selon le cas, permettant ainsi de séparer le premier PUFA d'impuretés qui sont plus ou moins retenues que lui.

La première unité chromatographique 10 est alimentée par une conduite d'alimentation en mélange d'acides gras 9 ainsi que par une conduite d'alimentation en éluant 11.

En sortie de la première unité chromatographique 10 sont connectées d'une part une conduite de flux enrichi en premier PUFA 12 et d'autre part une conduite de flux enrichi en deuxième acide gras 13.

Dans le contexte de la présente demande, le terme « *enrichi*» a une signification relative : une séparation entre une espèce A et une espèce B à partir d'un flux initial, permettant de récupérer un flux enrichi en espèce A, signifie ainsi que le flux ainsi récupéré présente un rapport massique A/B supérieur à celui du flux initial.

La conduite de flux enrichi en premier PUFA 12 alimente une unité de traitement 14, qui est adaptée à réaliser une réduction de l'indice de peroxyde et/ou de l'indice d'anisidine. En sortie de celle-ci est connectée une conduite de collecte de premier PUFA purifié 15.

Si le procédé de l'invention comporte une seule étape chromatographique, les autres parties de l'installation représentées sur la figure sont omises. Dans ce cas, la conduite d'alimentation en mélange d'acides gras 9 alimente la première unité chromatographique 10 avec le mélange initial (étant entendu que ce mélange initial peut avoir subi des étapes préliminaires de traitement telles que décrites ci-dessus, auquel cas les unités de traitement correspondantes, non représentées, peuvent être incluses dans l'installation).

Alternativement, et comme représenté sur la figure, une deuxième unité chromatographique 6 peut être prévue, pour assurer une séparation entre le premier PUFA et un troisième acide gras. Cette deuxième unité chromatographique 6 est alimentée par une conduite d'alimentation en mélange d'acides gras 5 ainsi que par une conduite d'alimentation en éluant 7.

En sortie de la deuxième unité chromatographique 6 sont connectées d'une part une conduite de flux enrichi en premier PUFA 9 et d'autre part une conduite de flux enrichi en troisième acide gras 8. La conduite de flux enrichi en premier PUFA 9 constitue la conduite d'alimentation en mélange d'acides gras 9 pour la première unité chromatographique 10.

Si le procédé de l'invention comporte deux étapes chromatographiques seulement, les autres parties de l'installation représentées sur la figure sont omises. Dans ce cas, la conduite d'alimentation en mélange d'acides gras 5 alimente la deuxième unité chromatographique 6 avec le mélange initial (étant entendu que ce mélange initial peut avoir subi des étapes préliminaires de traitement telles que décrites ci-dessus, auquel cas les unités de traitement correspondantes, non représentées, peuvent être incluses dans l'installation).

Alternativement, et comme représenté sur la figure, une troisième unité chromatographique 3 peut être prévue, pour assurer une séparation entre le premier PUFA et un quatrième acide gras. Cette troisième unité chromatographique 3 est alimentée par une conduite d'alimentation en mélange d'acides gras 1 ainsi que par une conduite d'alimentation en éluant 2.

En sortie de la troisième unité chromatographique 3 sont connectées d'une part une conduite de flux enrichi en premier PUFA 5 et d'autre part une conduite de flux enrichi en quatrième acide gras 4. La conduite de flux enrichi en premier PUFA 5 constitue la conduite d'alimentation en mélange d'acides gras 5 pour la deuxième unité chromatographique 6.

Si le procédé de l'invention comporte trois étapes chromatographiques, la conduite d'alimentation en mélange d'acides gras 1 alimente la troisième unité chromatographique 3 avec le mélange initial (étant entendu que ce mélange initial peut avoir subi des étapes préliminaires de traitement telles que décrites ci-dessus, auquel cas les unités de traitement correspondantes, non représentées, peuvent être incluses dans l'installation).

Alternativement, on peut prévoir encore d'autres étapes de séparation chromatographique analogues.

Ainsi, dans le mode de réalisation illustré, le mélange initial subit trois étapes successives de chromatographie en phase liquide :
- une étape permettant de séparer le premier PUFA du quatrième acide gras (dans la troisième unité chromatographique 3) ;
- une étape permettant de séparer le premier PUFA du troisième acide gras (dans la deuxième unité chromatographique 6) ;
- une étape permettant de séparer le premier PUFA du deuxième acide gras (dans la première unité chromatographique 10).

Alternativement, le mélange initial subit uniquement deux étapes successives de chromatographie en phase liquide :
- une étape permettant de séparer le premier PUFA du troisième acide gras (dans la deuxième unité chromatographique 6) ;
- une étape permettant de séparer le premier PUFA du deuxième acide gras (dans la première unité chromatographique 10).

Alternativement, le mélange initial subit une seule étape de chromatographie en phase liquide, à savoir la séparation du premier PUFA et du deuxième acide gras (dans la première unité chromatographique 10).

Alternativement, le mélange initial subit quatre étapes successives de chromatographie en phase liquide :
- une étape permettant de séparer le premier PUFA d'un cinquième acide gras (dans une unité chromatographique non représentée) ;
- une étape permettant de séparer le premier PUFA du quatrième acide gras (dans la troisième unité chromatographique 3) ;
- une étape permettant de séparer le premier PUFA du troisième acide gras (dans la deuxième unité chromatographique 6) ;
- une étape permettant de séparer le premier PUFA du deuxième acide gras (dans la première unité chromatographique 10).

### Nature des séparations chromatographiques

Le terme « *unité chromatographique »* désigne soit un système chromatographique à colonne unique soit un système chromatographique à plusieurs colonnes.

Des exemples de systèmes chromatographiques à colonne unique sont les systèmes HPLC (chromatographie en phase liquide à haute performance) ou CYCLOJET^{™} (système avec recyclage à l'état stationnaire). Des exemples de systèmes chromatographiques à plusieurs colonnes sont les systèmes SMB, iSMB, AMB, VARICOL^{™}, MODICON^{™}, POWERFEED^{™}, DCC, MCSGP ou GSSR (chromatographie de gradient multi-colonnes).

Le système CYCLOJET^{™} est tel que décrit dans le document US 6,063,284, auquel il est fait expressément référence. Il s'agit d'un système de séparation chromatographique discontinue à colonne unique, dans lequel les espèces (i) les plus retenues puis (ii) les moins retenues sont collectées séparément à la sortie de la colonne, une portion non-séparée du chromatogramme étant recyclée par une pompe principale. Le mélange à séparer est périodiquement injecté au moyen d'une boucle d'injection dans la portion recyclée du chromatogramme. La boucle d'injection est de préférence connectée entre la pompe principale et la colonne. Après plusieurs cycles chromatographiques, le procédé atteint un état stationnaire périodique dans lequel la quantité de produits injectés est égale à la quantité de produits collectés séparément à la sortie de la colonne.

Un système SMB comprend une pluralité de colonnes individuelles contenant un adsorbant, qui sont connectées en série. Un flux d'éluant traverse les colonnes selon une première direction. Les points d'injection du flux d'alimentation et de l'éluant, ainsi que les points de collecte des composés séparés, sont décalés périodiquement et simultanément au moyen d'un ensemble de vannes. L'effet global est de simuler le fonctionnement d'une colonne unique contenant un lit mobile d'adsorbant solide, l'adsorbant solide se déplaçant dans une direction à contre-courant du flux d'éluant. Ainsi, un système SMB est composé de colonnes qui contiennent des lits stationnaires d'adsorbant solide à travers lesquels passe l'éluant, mais le fonctionnement est tel qu'un lit mobile continu à contre-courant est simulé.

La forme la plus conventionnelle d'un système SMB est le système SMB à quatre zones. D'autres formes possibles sont les systèmes SMB à trois zones et les systèmes SMB à deux zones (tels que décrits dans l'article « Two Section Simulated Moving Bed Process » de Kwangnam Lee, dans Separation Science and Technology 35(4):519-534, 2000, auquel il est fait expressément référence).

Un système iSMB est tel que décrit dans les documents EP 0342629 et US 5,064,539, auxquels il est fait expressément référence. Un système SSMB découpe les introductions et collectes des flux en sous séquences appliquées de façons périodiques. Dans les systèmes iSMB et SSMB, il y a au moins une étape dans laquelle le système fonctionne en boucle fermée, sans entrée ou sortie de produit.

D'autres variantes des systèmes SMB sont : le système SMB variant dans le temps et le système POWERFEED^{™}, tels que décrits dans le document US 5,102,553 et dans l'article « *PowerFeed opération of simulated moving bed units: changing flow-rates during the switching interval*»*,* de Zhang et al. dans Journal of Chromatography A, 1006:87-99, 2003, auxquels il est fait expressément référence; le système MODICON^{™}, tel que décrit dans le document US 7,479,228, auquel il est fait expressément référence ; et le système SMB avec recirculation interne, tel que décrit dans le document US 8,282,831, auquel il est fait expressément référence.

Un système AMB présente un fonctionnement similaire à un système SMB. Toutefois, au lieu de déplacer les points d'injection du flux d'alimentation et de l'éluant, ainsi que des points de collecte, au moyen d'un système de vannes, un ensemble d'unités d'adsorption (colonnes) sont déplacées physiquement par rapport aux points d'alimentation et de collecte. A nouveau, le fonctionnement permet de simuler un lit mobile continu à contre-courant.

Un système de chromatographie VARICOL^{™} est tel que décrit dans les documents US 6,136,198, US 6,375,839 US 6,413,419 et US 6,712,973, auxquels il est fait expressément référence. Un système VARICOL^{™} comprend une pluralité de colonnes individuelles contenant un adsorbant qui sont reliées en série. On fait passer un éluant dans les colonnes selon une première direction. Contrairement au système SMB, les points d'injection pour le mélange à séparer et pour l'éluant et les points de collecte des composés séparés dans le système sont déplacés périodiquement mais de manière asynchrone, au moyen d'un ensemble de vannes. L'effet global est de créer des zones de séparation de longueur variable dans le temps, allouant ainsi la phase stationnaire de manière dynamique dans les zones où elle est la plus utile, et permettant une puissance de séparation similaire avec moins d'unités chromatographiques et une productivité accrue. Contrairement à un système SMB, un système VARICOL^{™}ne simule pas le fonctionnement d'une colonne unique contenant un lit mobile d'adsorbant solide, l'adsorbant solide se déplaçant dans une direction à contre-courant du flux d'éluant, et ainsi le principe de fonctionnement du VARICOL^{™}ne peut pas être mis en œuvre dans un système AMB équivalent.

Un système de chromatographie DCC est tel que décrit dans le document FR 2889077, auquel il est fait expressément référence. Un système DCC est un procédé séquentiel à déplacement périodique des points d'injection de phase mobile et de mélange à séparer, ayant la caractéristique d'être constamment en boucle ouverte. Il utilise deux colonnes ou plus.

Selon un mode de réalisation, le procédé de l'invention comprend deux étapes de séparation chromatographique successives (et deux seulement), qui peuvent être des étapes de séparation AMB, SMB ou VARICOL^{™}.

Selon un mode de réalisation, le procédé de l'invention comprend trois étapes de séparation chromatographiques successives (et trois seulement), qui peuvent être des étapes de séparation AMB, SMB ou VARICOL^{™}.

Selon un mode de réalisation, le procédé de l'invention comprend trois étapes de séparation chromatographiques successives (et trois seulement), avec d'abord une étape de séparation de type VARICOL^{™} (pour séparer le premier PUFA du quatrième acide gras), puis une étape de séparation de type CYCLOJET^{™} ou HPLC (pour séparer le premier PUFA du troisième acide gras), puis une étape de séparation de type VARICOL^{™} (pour séparer le premier PUFA du deuxième acide gras).

Selon un mode de réalisation, le procédé de l'invention comprend trois étapes de séparation chromatographiques successives (et trois seulement), avec d'abord une étape de séparation de type VARICOL^{™} (pour séparer le premier PUFA du quatrième acide gras), puis une étape de séparation de type CYCLOJET^{™} ou HPLC (pour séparer le premier PUFA du troisième acide gras), puis une étape de séparation de type CYCLOJET^{™} ou HPLC (pour séparer le premier PUFA du deuxième acide gras).

Lorsque le procédé comprend deux étapes de séparation chromatographique ou davantage, ces étapes peuvent être effectuées simultanément dans des unités physiquement distinctes (de même type ou de types et/ou de tailles différents), ou peuvent être effectuées séquentiellement, dans des unités physiquement distinctes ou dans des mêmes unités.

En outre, lorsque deux étapes de séparation chromatographique sont effectuées dans un système de type SMB ou AMB, il est possible de les mettre en œuvre simultanément sur un même système SMB ou AMB. Un exemple de mise en œuvre simultanée sur un même appareil est décrit dans le document WO 2011/080503, ou le document WO 2013/005048, ou le document WO 2013/005051, auxquels il est fait expressément référence.

Ainsi, certaines des unités de séparation peuvent être les mêmes. Par exemple la première unité chromatographique 10 et la deuxième unité chromatographique 6 peuvent être la même unité ; ou la troisième unité chromatographique 3 et la deuxième unité chromatographique 6 peuvent être la même unité ; ou la première unité chromatographique 10 et la troisième unité chromatographique 3 peuvent être la même unité ; ou la première unité chromatographique 10, la deuxième unité chromatographique 6 et la troisième unité chromatographique 3 peuvent être la même unité.

Alternativement, toutes les unités chromatographiques peuvent être distinctes.

Chaque étape de séparation chromatographique peut être effectuée sur une phase inversée, en tant qu'adsorbant (phase stationnaire). Par exemple, on peut utiliser des adsorbants basés sur des résines faiblement polaires ou des phases stationnaires à base de silice chimiquement modifiée avec des groupements organiques tels que des groupements alkyles (notamment en C4, C8, C18, C24, C30), phényles, ou autres.

Chaque étape de séparation chromatographique peut être effectuée en utilisant un éluant hydro-organique, c'est-à-dire un mélange d'un ou plusieurs solvants organiques avec de l'eau. De préférence, toutes les étapes de séparation chromatographique sont effectuées en utilisant des éluants hydro-organiques. Alternativement, il est possible de mettre en œuvre certaines étapes de séparation chromatographiques avec des éluants purement organiques.

Les solvants organiques utilisables dans le cadre de l'invention (notamment pour former les éluants hydro-organiques) sont par exemple les alcools tels que l'éthanol, le propanol, l'isopropanol et de manière davantage préférée le méthanol ; les cétones telles que l'acétone ou la méthyléthyl-cétone ; les nitriles tels que l'acétonitrile ; les esters tels que l'acétate de méthyle ou l'acétate d'éthyle ; les furanes tels que le tétrahydrofurane ; les éthers tels que le diéthylether or le méthyléthylether ; et les combinaisons de deux ou plus de deux solvants parmi ceux-ci. Le méthanol et l'acétone sont les solvants organiques préférés.

Chaque éluant hydro-organique est caractérisé par un rapport eau/organique, qui est le rapport volumique de l'eau par rapport au(x) solvant(s) organique(s) dans l'éluant.

Le rapport eau/organique de chaque éluant hydro-organique peut de préférence varier de 0,01 :99,99 à 30:70, et de préférence de 5:95 à 25:75.

Quand le procédé comporte au moins deux étapes de séparation chromatographique, celles-ci peuvent être effectuées avec des éluants ayant la même composition ou des compositions différentes.

Il est préféré d'utiliser des éluants ayant des compositions différentes, et en particulier ayant des rapports eau/organique différents, cela permettant d'ajuster la force éluante de l'éluant à chaque étape de séparation et donc d'obtenir la séparation de différents composés à chaque étape. On peut aussi souhaiter utiliser des éluants composés de différents solvants organiques dans les différentes étapes, afin d'ajuster la sélectivité chromatographique entre certaines espèces devant être séparées à chaque étape de séparation et ainsi obtenir la séparation de différents composés à chaque étape.

De préférence, la concentration massique du premier éluant en solvant(s) organique(s) est contrôlée à 2 % près, de préférence à 1 % près, ou à 0,5 % près, ou à 0,2 % près, ou à 0,1 % près ; le cas échéant de préférence la concentration massique du deuxième éluant en solvant(s) organique(s) est contrôlée à 2 % près, de préférence à 1 % près, ou à 0,5 % près, ou à 0,2 % près, ou à 0,1 % près ; le cas échéant de préférence la concentration massique du troisième éluant en solvant(s) organique(s) est contrôlée à 2 % près, de préférence à 1 % près, ou à 0,5 % près, ou à 0,2 % près, ou à 0,1 % près. Le contrôle de la composition des éluants est effectué assurant des apports d'eau et/ou de solvant(s) organiques afin de procéder aux ajustements nécessaires.

En sortie de la première unité chromatographique 10, le flux enrichi en premier PUFA peut être le raffinat, et le flux enrichi en deuxième acide gras peut être l'extrait ; ou inversement, le flux enrichi en premier PUFA peut être l'extrait, et le flux enrichi en deuxième acide gras peut être le raffinat.

En sortie de la deuxième unité chromatographique 6, le flux enrichi en premier PUFA peut être le raffinat, et le flux enrichi en troisième acide gras peut être l'extrait ; ou inversement, le flux enrichi en premier PUFA peut être l'extrait, et le flux enrichi en troisième acide gras peut être le raffinat.

En sortie de la troisième unité chromatographique 3, le flux enrichi en premier PUFA peut être le raffinat, et le flux enrichi en quatrième acide gras peut être l'extrait ; ou inversement, le flux enrichi en premier PUFA peut être l'extrait, et le flux enrichi en quatrième acide gras peut être le raffinat.

Selon un mode de réalisation particulier, le flux enrichi en premier PUFA en sortie de la troisième unité chromatographique 3 est le raffinat, le flux enrichi en premier PUFA en sortie de la deuxième unité chromatographique 6 est le raffinat et le flux enrichi en premier PUFA en sortie de la première unité chromatographique 10 est l'extrait.

Chaque flux (raffinat ou extrait) issu d'une étape de séparation chromatographique est généralement concentré de sorte à en éliminer l'éluant (solvants organiques et eau) ou à réduire la teneur massique du flux en solvants organiques et eau à un niveau de moins de 10 %, ou de moins de 5 %, ou de moins de 2 %, ou de moins de 1 %, ou de moins de 0,5 %, ou de moins de 0,1 %).

Ainsi, dans un mode de réalisation préféré, au moins une unité de concentration (non représentée sur la figure) est associée à chaque unité chromatographique 3, 6, 10.

En particulier, de préférence, le flux enrichi en premier PUFA collecté à l'issue de la séparation chromatographique entre le premier PUFA et le deuxième acide gras est un flux concentré (appauvri en éluant ou dépourvu ou essentiellement dépourvu de solvants organiques et d'eau) ; de même, le cas échéant, le flux enrichi en premier PUFA collecté à l'issue de la séparation chromatographique entre le premier PUFA et le troisième acide gras, et le flux enrichi en premier PUFA collecté à l'issue de la séparation chromatographique entre le premier PUFA et le quatrième acide gras sont des flux concentrés (appauvris en éluant ou dépourvus ou essentiellement dépourvus de solvants organiques et d'eau) ; éventuellement, le flux enrichi en deuxième acide gras, et le cas échéant le flux enrichi en troisième acide gras et le flux enrichi en quatrième acide gras sont des flux concentrés (appauvris en éluant ou dépourvus ou essentiellement dépourvus de solvants organiques et d'eau).

Dans chaque unité de concentration, l'éluant peut être évaporé et condensé, de sorte à le séparer du mélange d'acides gras. On peut utiliser par exemple un évaporateur à film tombant à recirculation, un évaporateur à flux montant, un évaporateur à film raclé, un évaporateur à couche mince, un évaporateur à thermosiphon, un évaporateur rotatif, une colonne à distiller, une colonne de rectification ou tout autre évaporateur ou combinaison d'évaporateurs permettant l'évaporation de l'éluant et la concentration des acides gras concentrés au fond de l'appareil. L'évaporation est de préférence effectuée à une pression inférieure à la pression atmosphérique, notamment à une pression inférieure ou égale à 750 mbar, ou inférieure ou égale à 500 mbar, ou inférieure ou égale à 300 mbar.

Alternativement, on peut utiliser un dispositif de séparation membranaire, avec un ou plusieurs étages de séparation, ou une combinaison de moyens d'évaporation et de séparation membranaire.

L'éluant évaporé et condensé, ou autrement séparé, peut être recyclé vers une ou plusieurs étapes du procédé, notamment une ou plusieurs des étapes de séparation chromatographique.

Selon un mode de réalisation, chaque étape de concentration (et notamment chaque étape de concentration de flux enrichi en premier PUFA) peut être effectuée à une température inférieure ou égale à 120°C, de préférence inférieure ou égale à 100°C, de préférence inférieure ou égale à 90°C, de préférence inférieure ou égale à 80°C, inf érieure ou égale à 75°C.

Selon un mode de réalisation, chaque étape de concentration (et notamment chaque étape de concentration de flux enrichi en premier PUFA) présente une durée inférieure ou égale à 6 heures, de préférence inférieure ou égale à 4 heures, de préférence inférieure ou égale à 3 heures, de préférence inférieure ou égale à 2 heures, de préférence inférieure ou égale à 1 h30, de préférence inférieure ou égale à 1 heure.

Selon des modes de réalisation particuliers, l'éluant séparé à partir du premier flux enrichi en premier PUFA est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du deuxième flux enrichi en premier PUFA est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du troisième flux enrichi en premier PUFA est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du flux enrichi en deuxième acide gras est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du flux enrichi en troisième acide gras est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du flux enrichi en quatrième acide gras est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99%.

De préférence, le produit d'alimentation qui est fourni en entrée de chaque unité de séparation chromatographique et qui est destiné à être séparé est aussi dépourvu de solvants que possible.

Ainsi :
- le mélange initial comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques ; et/ou
- le cas échéant, le premier flux enrichi en premier PUFA qui alimente la deuxième unité de séparation chromatographique 6 comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques ; et/ou
- le deuxième flux enrichi en premier PUFA qui alimente la troisième unité de séparation chromatographique 10 comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques.

### Elimination des composés oxygénés

Le procédé selon l'invention prévoit une étape d'élimination (ou de réduction de la quantité) des composés oxygénés après la séparation chromatographique, ou après les séparations chromatographiques.

De préférence, cette étape n'est pas une étape de séparation chromatographique, et n'est pas mise en œuvre dans une unité chromatographique.

De préférence, cette étape ne sépare pas le premier PUFA d'autres acides gras présents dans le flux (à l'exception des composés oxygénés du type aldéhydes et peroxydes).

L'étape d'élimination des composés oxygénés est mise en œuvre dans l'unité de traitement 14. Cette unité de traitement 14 peut notamment être une unité de distillation moléculaire ou évaporateur à court trajet. Un évaporateur à court trajet est équipé d'un condenseur intérieur et peut produire des évaporations avec un temps de résidence de préférence inférieur à 1000 s, de préférence inférieur à 100 s, de préférence inférieur à 10 s, sous une pression préférence inférieure à 10 mbar, de préférence inférieure à 1 mbar, de préférence inférieure à 0,1 mbar, de préférence inférieure à 0,01 mbar, de préférence inférieure à 0,001 mbar, à une température inférieure ou égale à 200°C, de préfére nce inférieure ou égale à 150°C, de préférence inférieure ou égale à 120°C, d e préférence inférieure ou égale à 100°C, de préférence inférieure ou égale à 80°C.

Alternativement l'unité de traitement 14 peut être une unité de mise en contact avec un substrat d'adsorption.

Le substrat d'adsorption est tout substrat susceptible d'adsorber des composés oxygénés tels que les peroxydes et les composés aldéhydiques. Il peut être choisi par exemple parmi la silice, l'alumine, le charbon actif et les dérivés de ceux-ci, notamment les gels de silice, les silicates, aluminates et aluminosilicates. Une argile telle que la bentonite est un exemple de substrat approprié, de même que la terre de diatomée.

L'adsorption peut être effectuée en discontinu, c'est-à-dire en « *batch* », ou en continu, par percolation à travers un lit d'adsorbant. De préférence, les acides gras ne sont pas dilués au cours de cette étape, et en particulier aucun solvant n'est ajouté. La mise en contact peut durer par exemple de 5 minutes à 24 heures, et notamment de 10 minutes à 10 heures, de 20 minutes à 5 heures, de 30 minutes à 2 heures, et de 45 minutes à 1 h30.

La quantité d'adsorbant utilisée dépend de la nature de l'adsorbant et de sa capacité à capturer les composés oxygénés. Elle peut être par exemple de 1 à 1000 g d'adsorbant par kg de flux à traiter (mélange d'acides gras), notamment de 10 à 500 g/kg, et plus particulièrement de 25 à 200 g/kg.

A l'issue de la mise en contact, l'adsorbant est séparé du mélange d'acides gras, et ce dernier peut être filtré afin d'éviter toute contamination par de l'adsorbant résiduel.

De préférence, la liaison des composés oxygénés à l'adsorbant est essentiellement irréversible, c'est-à-dire que l'adsorbant n'est pas régénéré.

Il est cependant possible de régénérer l'adsorbant, par traitement thermique par exemple, afin de limiter le volume et/ou le coût de traitement des déchets.

L'étape de traitement dans l'unité de traitement 14 peut permettre de réduire l'indice de peroxyde du flux traité d'au moins 25 %, de préférence d'au moins 50 %, de préférence d'au moins 75 %, de préférence d'au moins 80 % ou d'au moins 90 % ou d'au moins 95 % ou d'au moins 98 %.

L'étape de traitement dans l'unité de traitement 14 peut permettre de réduire l'indice d'anisidine du flux traité d'au moins 25 %, de préférence d'au moins 50 %, de préférence d'au moins 75 %, de préférence d'au moins 80 % ou d'au moins 90 % ou d'au moins 95 % ou d'au moins 98 %.

L'indice de peroxyde mesure la quantité de composés peroxydes dans un mélange d'acides gras. La méthode d'analyse utilisée est de préférence Ph Eur 2.5.5 met A.

L'indice d'anisidine mesure la quantité de composés aldéhydiques dans un mélange d'acides gras. La méthode d'analyse utilisée est de préférence Ph Eur 2.5.36.

Selon un mode de réalisation, l'indice de peroxyde du flux issu de l'étape de traitement (produit récolté dans la conduite de collecte de premier PUFA purifié 15) est inférieur ou égal à 10, ou inférieur ou égal à 9, ou inférieur ou égal à 8, ou inférieur ou égal à 7, ou inférieur ou égal à 6, ou inférieur ou égal à 5, ou inférieur ou égal à 4, ou inférieur ou égal à 3, ou inférieur ou égal à 2, ou inférieur ou égal à 1,5, ou inférieur ou égal à 1.

Selon un mode de réalisation, l'indice d'anisidine du flux issu de l'étape de traitement (produit récolté dans la conduite de collecte de premier PUFA purifié 15) est inférieur ou égal à 20, ou inférieur ou égal à 18, ou inférieur ou égal à 16, ou inférieur ou égal à 14, ou inférieur ou égal à 12, ou inférieur ou égal à 10, ou inférieur ou égal à 9, ou inférieur ou égal à 8, ou inférieur ou égal à 7, ou inférieur ou égal à 6, ou inférieur ou égal à 5, ou inférieur ou égal à 4, ou inférieur ou égal à 3, ou inférieur ou égal à 2.

Une autre étape de traitement analogue à celle décrite ci-dessus, et plus particulièrement une étape de distillation moléculaire, peut également être prévue en amont, notamment avant toute étape de séparation chromatographique.

Ainsi, selon un mode de réalisation, l'indice de peroxyde du mélange initial qui est soumis à la ou aux séparations chromatographiques est inférieur ou égal à 10, ou inférieur ou égal à 9, ou inférieur ou égal à 8, ou inférieur ou égal à 7, ou inférieur ou égal à 6, ou inférieur ou égal à 5, ou inférieur ou égal à 4, ou inférieur ou égal à 3, ou inférieur ou égal à 2, ou inférieur ou égal à 1,5, ou inférieur ou égal à 1.

Selon un mode de réalisation, l'indice d'anisidine du mélange initial qui est soumis à la ou aux séparations chromatographiques est inférieur ou égal à 20, ou inférieur ou égal à 18, ou inférieur ou égal à 16, ou inférieur ou égal à 14, ou inférieur ou égal à 12, ou inférieur ou égal à 10, ou inférieur ou égal à 9, ou inférieur ou égal à 8, ou inférieur ou égal à 7, ou inférieur ou égal à 6, ou inférieur ou égal à 5, ou inférieur ou égal à 4, ou inférieur ou égal à 3, ou inférieur ou égal à 2.

Les inventeurs ont constaté que l'indice de peroxyde et/ou l'indice d'anisidine augmentent au cours des étapes de séparation chromatographiques décrites ci-dessus, lorsque le premier PUFA est séparé de composés plus apolaires que lui, et ce bien que les conditions de séparation chromatographique soient des conditions douces - les séparations chromatographiques étant notamment effectuées en l'absence de dioxygène et à l'abri de la lumière, et/ou à une température modérée. Sans vouloir être liés par une théorie, les inventeurs estiment que cette augmentation peut être due notamment à l'utilisation d'éluants hydro-organiques.

Selon un mode de réalisation, l'indice de peroxyde du flux contenant le premier PUFA augmente lors de l'étape de séparation chromatographique entre le premier PUFA et le deuxième acide gras (c'est-à-dire entre la conduite de conduite d'alimentation en mélange d'acides gras 9 et la conduite de conduite de flux enrichi en premier PUFA 12), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Selon un mode de réalisation, l'indice d'anisidine du flux contenant le premier PUFA augmente lors de l'étape de séparation chromatographique entre le premier PUFA et le deuxième acide gras (c'est-à-dire entre la conduite de conduite d'alimentation en mélange d'acides gras 9 et la conduite de conduite de flux enrichi en premier PUFA 12), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Selon un mode de réalisation, l'indice de peroxyde du flux contenant le premier PUFA augmente lors de l'étape de séparation chromatographique entre le premier PUFA et le troisième acide gras (c'est-à-dire entre la conduite de conduite d'alimentation en mélange d'acides gras 5 et la conduite de conduite de flux enrichi en premier PUFA 9), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Selon un mode de réalisation, l'indice d'anisidine du flux contenant le premier PUFA augmente lors de l'étape de séparation chromatographique entre le premier PUFA et le troisième acide gras (c'est-à-dire entre la conduite de conduite d'alimentation en mélange d'acides gras 5 et la conduite de conduite de flux enrichi en premier PUFA 9), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Selon un mode de réalisation, l'indice de peroxyde du flux contenant le premier PUFA augmente lors de l'étape de séparation chromatographique entre le premier PUFA et le quatrième acide gras (c'est-à-dire entre la conduite de conduite d'alimentation en mélange d'acides gras 1 et la conduite de conduite de flux enrichi en premier PUFA 5), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Selon un mode de réalisation, l'indice d'anisidine du flux contenant le premier PUFA augmente lors de l'étape de séparation chromatographique entre le premier PUFA et le quatrième acide gras (c'est-à-dire entre la conduite de conduite d'alimentation en mélange d'acides gras 1 et la conduite de conduite de flux enrichi en premier PUFA 5), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Selon un mode de réalisation, l'indice de peroxyde du flux enrichi en premier PUFA 13 issu de la séparation chromatographique du premier PUFA et du deuxième acide gras (c'est-à-dire issu de la première unité chromatographique 10) est supérieur à l'indice de peroxyde du mélange initial (c'est-à-dire le mélange éventuellement prétraité qui est destiné à être soumis à la ou aux étapes de séparation chromatographique), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Selon un mode de réalisation, l'indice d'anisidine du flux enrichi en premier PUFA 13 issu de la séparation chromatographique du premier PUFA et du deuxième acide gras (c'est-à-dire issu de la première unité chromatographique 10) est supérieur à l'indice d'anisidine du mélange initial (c'est-à-dire mélange éventuellement prétraité qui est destiné à être soumis à la ou aux étapes de séparation chromatographique), de préférence d'au moins 30 %, ou d'au moins 50 %, ou d'au moins 75 %, ou d'au moins 100 %, ou d'au moins 150 %, ou d'au moins 200 %, ou d'au moins 300 %, ou d'au moins 500 %.

Dans l'hypothèse où l'une des séparations chromatographiques effectuées n'a pas pour conséquence d'augmenter significativement l'indice de peroxyde ou l'indice d'anisidine du flux d'intérêt, il est possible d'inverser l'ordre des étapes, c'est-à-dire d'effectuer cette ou ces étapes de séparation chromatographique après l'étape de réduction de l'indice de peroxyde / de l'indice d'anisidine.

Ainsi, dans ce cas de figure, on peut envisager par exemple :
- une étape de séparation chromatographique du premier PUFA et du deuxième acide gras, suivie d'une étape de traitement comprenant la réduction de l'indice de peroxyde et/ou de l'indice d'anisidine du flux enrichi en premier PUFA, suivie d'une étape de séparation chromatographique du premier PUFA et du troisième acide gras ; ou
- une étape de séparation chromatographique du premier PUFA et du troisième acide gras, suivie d'une étape de séparation chromatographique du premier PUFA et du deuxième acide gras, suivie d'une étape de traitement comprenant la réduction de l'indice de peroxyde et/ou de l'indice d'anisidine du flux enrichi en premier PUFA, suivie d'une étape de séparation chromatographique du premier PUFA et du quatrième acide gras ; ou
- une étape de séparation chromatographique du premier PUFA et du deuxième acide gras, suivie d'une étape de traitement comprenant la réduction de l'indice de peroxyde et/ou de l'indice d'anisidine du flux enrichi en premier PUFA, suivie d'une étape de séparation chromatographique du premier PUFA et du quatrième acide gras, suivie d'une étape séparation chromatographique du premier PUFA et du troisième acide gras.

Le procédé peut comprendre une étape d'ajout d'un stabilisant (antioxydant), tel que le tocophérol, l'acide ascorbique ou tout autre composé ou mélange de composés connu de l'homme de l'art, afin d'éviter une dégradation du produit et une augmentation supplémentaire de l'indice de peroxyde ou d'anisidine. Cette étape d'ajout de stabilisant est de préférence effectuée à la fin du procédé, après toutes les étapes de séparation chromatographique et après l'étape de traitement.

### Produit obtenu

Selon un mode de réalisation, le premier PUFA est un acide gras omega-3.

Selon des modes de réalisation différents, le premier PUFA peut être l'EPA, ou le DHA, ou l'ARA, ou le DPA, ou le SDA.

Selon un mode de réalisation, le premier PUFA est l'EPA et le deuxième acide gras est le DHA.

Selon un mode de réalisation, le premier PUFA est le DHA et le deuxième acide gras est l'EPA.

Selon un mode de réalisation, le premier PUFA est l'EPA et le deuxième acide gras est le SDA.

Selon un mode de réalisation, le premier PUFA est le SDA et le deuxième acide gras est l'EPA.

Selon un mode de réalisation, le premier PUFA est le DPA et le deuxième acide gras est le DHA.

Selon un mode de réalisation, le premier PUFA est le DHA et le deuxième acide gras est le DPA.

Selon un mode de réalisation, le premier PUFA est l'ARA et le deuxième acide gras est le DHA.

Selon un mode de réalisation, le premier PUFA est le DHA et le deuxième acide gras est l'ARA.

Selon un mode de réalisation, le premier PUFA est l'EPA, le deuxième acide gras est le DHA ou le SDA, et le troisième acide gras et le quatrième acide gras sont choisis parmi celui du DHA et du SDA qui n'est pas le deuxième acide gras, et parmi les acides gras saturés et les acides gras mono-insaturés.

La concentration en premier PUFA dans le produit final obtenu à l'issue du procédé (récolté dans la conduite de collecte de premier PUFA purifié 15) peut être supérieure ou égale à environ 80 %, de préférence supérieure ou égale à environ 90%, ou à environ 95 %, ou à environ 97 %, ou à environ 98 %, ou à environ 99 % (par rapport au total des acides gras).

La concentration en deuxième acide gras dans ce produit final peut être inférieure ou égale à environ 1 %, ou à environ 0,1%, ou à environ 0,05 %, ou à environ 0,03 %, ou à environ 0,01% (par rapport au total des acides gras).

La concentration en troisième acide gras dans ce produit final peut être inférieure ou égale à environ 1 %, ou à environ 0,1%, ou à environ 0,05 %, ou à environ 0,03 %, ou à environ 0,01% (par rapport au total des acides gras).

La concentration en quatrième acide gras dans ce produit final peut être inférieure ou égale à environ 1 %, ou à environ 0,1%, ou à environ 0,05 %, ou à environ 0,03 %, ou à environ 0,01% (par rapport au total des acides gras).

Par exemple, le produit final obtenu à l'issue du procédé (récolté dans la conduite de collecte de premier PUFA purifié 15), peut contenir de l'EPA dans une proportion supérieure ou égale à environ 80 %, ou supérieure ou égale à environ 95 %, ou supérieure ou égale à environ 97 %, ou supérieure ou égale à environ 98 %, ou supérieure ou égale à environ 99% (par rapport au total des acides gras); ainsi que du DHA dans une proportion inférieure ou égale à environ 1 %, ou inférieure ou égale à environ 0,1%, ou inférieure ou égale à environ 0,05 %, ou inférieure ou égale à environ 0,03 %, ou inférieure ou égale à environ 0,01%.

L'huile enrichie en premier PUFA est de préférence stockée à l'abri de l'air et de la lumière avant son conditionnement et/ou son utilisation, comprenant par exemple la formulation finale et/ou l'encapsulation.

Selon un mode de réalisation, le produit final est combiné avec un véhicule pharmaceutiquement et/ou diététiquement acceptable et/ou des excipients et/ou diluants. Ce produit peut ainsi être formulé par exemple sous forme de gélules, capsules ou comprimés (ou sous toute autre forme adaptée à une administration orale ou topique ou parentérale).

Chaque forme de dosage individuelle (par exemple capsule ou gélule) peut contenir par exemple de 250 à 1500 mg, de préférence de 300 à 1000 mg du produit ci-dessus.

Le produit peut ainsi être utilisé pour la préparation d'une composition pharmaceutique pour la prévention et/ou le traitement et/ou la prophylaxie de facteurs de risques pour les maladies cardiovasculaires, comme l'hypertriglycéridémie, l'hypercholestérolémie et l'hypertension ; et de maladies cardiovasculaires comme l'arythmie, la fibrillation atriale et/ou ventriculaire, la décompensation et l'insuffisance cardiaque ; pour la prévention primaire et secondaire de l'infarctus et du ré-infarctus ; pour le traitement de toute autre pathologie pouvant être traitée par les PUFA susmentionnés, comme par exemple les maladies auto-immunes, la cholique ulcéreuse, les pathologies tumorales, les maladies du système nerveux, le vieillissement cellulaire, l'infarctus cérébral, les maladies ischémiques, le psoriasis.

Alternativement, le produit peut être utilisé dans des utilisations parapharmaceutiques, et notamment diététiques, en particulier dans la nutrition infantile.

### EXEMPLES

L'exemple suivant illustre l'invention sans la limiter.

Dans cet exemple, un ester éthylique d'EPA d'une pureté de plus de 96 % est obtenu par trois étapes de chromatographie successives, à partir d'un mélange d'esters éthyliques contenant plus de 50 % d'EPA, et ayant un indice de peroxyde de 4,5 et un indice d'anisidine de 11.

L'ensemble des opérations de chromatographie sont réalisées sous atmosphère inerte et à l'abri de la lumière.

L'ensemble des opérations de chromatographie sont réalisées sur un système chromatographique de type VARICOL^{™}, comportant cinq colonnes de 20 cm de diamètre, remplies d'une phase stationnaire de silice phase inversée en C18. Le système chromatographie est couplé à deux évaporateurs à film tombant à recirculation, pour la concentration de l'extrait et du raffinat, respectivement.

Les conditions de chromatographie et d'évaporation sont les suivantes :
Etape 1 :
   - éluant : acétone/eau dans un rapport 90/10 v/v ;
   - évaporation complète de l'éluant du raffinat (produit cible) à 250 mbar et 75°C ;
   - évaporation complète de l'éluant de l'extrait à 250 mbar et 75°C.

Le raffinat concentré contient de l'EPA à environ 70 % (mesure de l'aire par chromatographie en phase gazeuse) et moins de 1% de solvants résiduels. L'indice de peroxyde moyen obtenu est de 8,0 et l'indice d'anisidine moyen obtenu est de 13,8.

Etape 2 :
- éluant : méthanol/eau dans un rapport 93/7 v/v ;
- évaporation complète de l'éluant du raffinat (produit cible) à 250 mbar et 75°C ;
- évaporation partielle de l'éluant de l'extrait à 1000 mbar et 75°C.

Le raffinat concentré contient de l'EPA à environ 92 % (mesure de l'aire par chromatographie en phase gazeuse) et moins de 1 % de solvants résiduels. L'indice de peroxyde moyen obtenu est de 7,4 et l'indice d'anisidine moyen obtenu est de 22,6.

Etape 3 :
- éluant : acétone/eau dans un rapport 79/21 v/v ;
- évaporation complète de l'éluant du raffinat à 250 mbar et 75°C ;
- évaporation complète de l'éluant de l'extrait (produit cible) à 250 mbar et 75°C.

L'extrait concentré contient de l'EPA à environ 97 % (mesure de l'aire par chromatographie en phase gazeuse) et moins de 1 % de solvants résiduels. L'indice de peroxyde moyen obtenu est de 6,5 et l'indice d'anisidine moyen obtenu est de 6,1.

Une étape finale d'entraînement à l'azote (« *stripping* ») et d'élimination des composés d'oxydation est réalisée en discontinu (« *batch* ») dans les conditions suivantes :
- stripping par bullage d'azote à 75°C pendant 1 he ure ;
- ajout 100 g de de bentonite par kg d'huile, agitation sous vide à 50 mbar pendant 1 heure ;
- filtration.

Un antioxydant (tocophérol 0,2 %) est ajouté. L'indice de peroxyde et l'indice d'anisidine dans l'EPA purifié sont mesurés entre 0,6 et 0,7 et entre 1,0 et 2,6 respectivement.

## Revendications

1. Installation de purification d'un premier acide gras polyinsaturé à partir d'un mélange initial, l'installation comprenant :
- au moins une unité de séparation chromatographique en phase liquide (10) du premier acide gras polyinsaturé et d'un deuxième acide gras, à laquelle sont connectées en sortie d'une part une conduite de flux enrichi en premier acide gras polyinsaturé (12) et d'autre part une conduite de flux enrichi en deuxième acide gras (13) ;
- au moins une unité de traitement (14) alimentée par la conduite de flux enrichi en premier acide gras polyinsaturé (13), l'unité de traitement (14) étant adaptée à effectuer une diminution de l'indice de peroxyde et / ou l'indice d'anisidine, dans laquelle l'unité de traitement (14) est une unité de distillation moléculaire ou est une unité de mise en contact avec un substrat d'adsorption, ladite unité de mise en contact ne comportant pas d'apport de solvant.

2. Installation selon la revendication 1, dans laquelle le substrat d'adsorption est choisi parmi la silice, l'alumine, le charbon actif et les dérivés de ceux-ci.

3. Installation selon la revendication 1 ou 2, comprenant une unité de séparation chromatographique en phase liquide (6) du premier acide gras polyinsaturé et d'un troisième acide gras, et éventuellement une unité de séparation chromatographique en phase liquide (3) du premier acide gras polyinsaturé et d'un quatrième acide gras, lesdites unités de séparation chromatographique en phase liquide (3, 6) étant de préférence situées en amont de l'unité de séparation chromatographique en phase liquide (10) du premier acide gras polyinsaturé et d'un deuxième acide gras.

4. Installation selon l'une des revendications 1 à 3, dans laquelle au moins une unité de séparation chromatographique en phase liquide (3, 6, 10), de préférence toutes les unités de séparation chromatographique en phase liquide (3, 6, 10), sont des unités de séparation chromatographique comportant une pluralité de colonnes de séparation chromatographique, et de préférence sont des unités de séparation chromatographique en lit mobile simulé et / ou en lit mobile réel.

## Patentansprüche

1. Anlage zur Reinigung einer ersten mehrfach ungesättigten Fettsäure ausgehend von einer anfänglichen Mischung, wobei die Anlage Folgendes umfasst:
- mindestens eine Einheit zur chromatographischen Trennung in flüssiger Phase (10) der ersten mehrfach ungesättigten Fettsäure und einer zweiten Fettsäure, mit der am Ausgang einerseits eine Flussleitung, angereichert mit einer ersten mehrfach ungesättigten Fettsäure (12) und andererseits eine Flussleitung, angereichert mit einer zweiten Fettsäure (13), verbunden ist;
- mindestens eine Bearbeitungseinheit (14), versorgt durch die Flussleitung, angereichert durch eine erste mehrfach ungesättigte Fettsäure (13), wobei die Bearbeitungseinheit (14) ausgelegt ist, um eine Verkleinerung des Peroxidindexes und/oder Anisidinindexes zu bewirken, wobei die Bearbeitungseinheit (14) eine Einheit zur molekularen Destillation ist oder eine Einheit zum In-Kontakt-Bringen mit einem Adsorptionssubstrat ist, wobei die Einheit zum In-Kontakt-Bringen keinen Lösemitteleintrag umfasst.

2. Anlage nach Anspruch 1, wobei das Adsorptionssubstrat ausgewählt ist aus Silizium, Aluminiumoxid, Aktivkohle und den Derivaten dieser.

3. Anlage nach Anspruch 1 oder 2, umfassend eine Einheit zur chromatographischen Trennung in flüssiger Phase (6) der ersten mehrfach ungesättigten Fettsäure und einer dritten Fettsäure, und eventuell eine Einheit zur chromatographischen Trennung in flüssiger Phase (3) der ersten mehrfach ungesättigten Fettsäure und einer vierten Fettsäure, wobei sich die Einheiten zur chromatographischen Trennung in flüssiger Phase (3 ,6) vorzugsweise vorgelagert von der Einheit zur chromatographischen Trennung in flüssiger Phase (10) der ersten mehrfach ungesättigten Fettsäure und einer zweiten Fettsäure befinden.

4. Anlage nach einem der Ansprüche 1 bis 3, wobei mindestens eine Einheit zur chromatographischen Trennung in flüssiger Phase (3, 6, 10), vorzugsweise alle Einheiten zur chromatographischen Trennung in flüssiger Phase (3, 6, 10) Einheiten zur chromatographischen Trennung sind, die eine Vielzahl von Säulen zur chromatographischen Trennung umfassen und vorzugsweise Einheiten zur chromatographischen Trennung in simuliertem Wanderbett und/oder in reellem Wanderbett Bett sind.

## Claims

1. An installation for purifying a first polyunsaturated fatty acid from an initial mixture, the installation comprising:
- at least one liquid chromatography unit (10) to separate a first polyunsaturated fatty acid and a second fatty acid, at the outlet of which there are connected a flow line enriched with first polyunsaturated fatty acid (12) and a flow line enriched with second fatty acid (13);
- at least one processing unit (14) fed by the flow line enriched with first polyunsaturated fatty acid (13), the processing unit (14) being adapted to carry out a decrease in peroxide value and/or anisidine value, wherein the processing unit (14) is a molecular distillation unit or a contacting unit with an adsorption substrate, said contacting unit not comprising a solvent supply.

2. The installation according to claim 1, wherein the adsorption substrate is selected from among silica, alumina, activated carbon and derivatives thereof.

3. The installation according to claim 1 or 2, comprising a liquid chromatography unit (6) to separate the first polyunsaturated fatty acid and a third fatty acid, and optionally a liquid chromatography unit (3) to separate the first polyunsaturated fatty acid and a fourth fatty acid, said liquid chromatography separation units (3,6) preferably being positioned upstream of the liquid chromatography unit (10) separating the first polyunsaturated fatty acid and a second fatty acid.

4. The installation according to one of claims 1 to 3, wherein at least one liquid chromatography separating unit (3, 6, 10), preferably all the liquid chromatography separation units (3, 6, 10) are chromatographic separation units comprising a plurality of chromatographic separation columns, and preferably they are simulated moving bed and/or true moving bed chromatographic separation units
